# EUROPEAN PATENT SPECIFICATION

(11) **EP 0 324 475 B1**
(45) Date of publication and mention of the grant of the patent: **07.01.1993**
(21) Application number: 89100483.0
(22) Date of filing: 12.01.1989
(51) Int. Cl.: C07C 43/04, C07C 41/01

(54) **One-step process for dimethyl ether synthesis utilizing a liquid phase reactor system**
Einstufiges Verfahren zur Synthese von Dimethyläther unter Verwendung eines Flüssigphasen-Reaktorsystems
Procédé en une seule opération pour la synthèse du diméthyl-éther utilisant un système de réacteur en phase liquide

(30) Priority: 14.01.1988 US 143799
(43) Date of publication of application: 19.07.1989
(73) Proprietor: AIR PRODUCTS AND CHEMICALS, INC., Trexlertown, PA 18087 (US)
(72) Inventor: Hsiung, Thomas Hsiao-Ling, Emmaus, PA 18049 (US); White, James Ferguson, Macungie, PA 18062 (US); Lewnard, John Joseph, Emmaus, PA 18049 (US)
(74) Representative: Sandmair, Kurt, Dr. Dr.

(56) References cited:
- DE-A- 3 118 620
- DE-A- 3 220 547
- GB-A- 278 353
- US-A- 4 341 069
- US-A- 4 417 000
- US-A- 4 423 155
- US-A- 4 481 305
- US-A- 4 520 216

## Description

The present invention relates to a process for the production of dimethyl ether. More specifically, the present invention relates to a process for the direct production of dimethyl ether from synthesis gas using a three-phase reactor system.

### BACKGROUND OF THE INVENTION

Conversion of synthesis gas to dimethyl ether requires three steps. Conventionally, synthesis gas is produced by reforming hydrocarbon or gasifying a carbon source such as coal or coke. Since this later synthesis gas usually is too rich in CO to be used directly for dimethyl ether synthesis, an intermediate step is needed for conventional dimethyl ether manufacture. Consequently, the first step in the dimethyl ether synthesis is to adjust the composition of the synthesis gas via the water-gas shift reaction:

CO + H₂O ⇄ CO₂ + H₂ (1)

After the ratio of hydrogen to carbon oxides has been adjusted, the gas is reacted to produce methanol (MeOH):

CO + 2 H₂ ⇄ CH₃OH (2)

Finally, methanol is dehydrated to form dimethyl ether (DME):

2 (CH₃OH) → CH₃OCH₃ + H₂O (3)

The shift and methanol synthesis reactions are equilibrium limited and exothermic. Moreover, the catalysts for both reactions are subject to severe deactivation when overheated. To avoid thermodynamic limitations and excessive catalyst deactivation, conventional gas phase reactors must be run at low per-pass conversions to maintain reactor temperature. Consequently, overall conversion of carbon monoxide to dimethyl ether is limited.

Multi-step processes, which use separate reactors for each reaction, can not exploit the potential synergism of the three reactions. If these three reactions could be conducted simultaneously, methanol synthesis would drive the forward shift reaction, and dimethyl ether synthesis would drive both the methanol and shift reaction. Consequently, a one-step process is more flexible and can operate under a wider range of conditions than a multi-step process. In addition, multi-step processes require separate reactors, heat exchangers, and associated equipment for each reaction.

A single-step gas phase process would generally require less equipment than multi-step gas processes. However, a single-step gas-phase process would still suffer from a large reactor exotherm due to the high net heat of reaction. Hence, low per-pass conversions would be required to maintain reactor temperature to avoid a short catalyst life due to the large temperature rises associated with these reactions. Since the reactor is not isothermal, there are often severe equilibrium limitations on per pass reactant conversions.

Much of the prior art for dimethyl ether synthesis focuses on processes using improved catalysts to run syngas having H₂/CO ratios greater than or equal to 1. Examples include U. S. Patents 4,417,000; 4,520,216; 4,590,176; and 4,521,540. These processes all run in the gas phase, and may be considered multi-step processes if they require that the feed be shifted via Reaction (1).

U. S. Patent 4,423,155 discloses a two-component catalyst for direct conversion of synthesis gas into dimethyl ether in a single gas-phase reactor. The synthesis gas may have a H₂/CO ratio within the range of 0.4-3.0.

Single-step gas-phase processes have been disclosed by Mobil Corp. and Haldor-Topsoe. For example, U.S. Patent 4,011,275 assigned to Mobil Corp. discloses a gas-phase process for co-production of methanol and dimethyl ether with H₂ deficient syngas feeds. Although there are no examples in the patent, the process is claimed to be useful for improving conversion of synthesis gas. U.S. Patent 4,341,069 discloses a gas-phase process for dimethyl ether production to be used in conjunction with an integrated gasification combined cycle power plant. Examples in the patent show that the catalyst requires frequent regeneration, in some cases on a daily basis. Another gas-phase process is described in U.S. Patent 4,481,305, however, this process is restricted to operation within a narrow range of CO/CO₂ ratio in the feed gas. It should be noted that efficient heat removal to maintain reactor temperature is generally not discussed in these patents. Fujimoto et al discussed in Chem. Letters, p.2051 (1984) the chemistry of the gas-phase one-step processes.

Combined methanol/dimethyl ether synthesis in the liquid phase has been reported by several workers. Sherwin and Blum, in their paper: "Liquid Phase Methanol Interim Report, May 1978", prepared for the Electric Power Research Institute, attempted to modify the liquid phase methanol process for co-production of dimethyl ether by adding acid catalyst components to the system. The observed only traces of dimethyl ether, and concluded that the attempt was unsuccessful. Daroda, et al, J.C.S. Chem. Comm. p.1101 (1980), reported a broad slate of products for reactions of syngas with Fe in 2-methoxyethanol. However, in their system the solvent appears to act as a reactant, and the catalyst produces many side products. Consequently, neither earlier liquid phase process was economic.

### SUMMARY OF THE INVENTION

The present invention is a single step process for direct production of dimethyl ether, with varying amounts of co-product methanol, from wide varieties of synthesis gas. Basically, the invention is an improvement to a process for the synthesis of dimethyl ether from a synthesis gas comprising hydrogen, carbon monoxide and carbon dioxide, wherein the synthesis gas is contacted and reacted in the presence of a solid catalyst. The improvement is the contacting and reacting of the synthesis gas in the presence of a solid catalyst system suspended in a liquid medium in a three phase (liquid phase) reactor system. The solid catalyst system can either be a single catalyst or a mixture of catalysts, provided the catalyst system comprises both a methanol synthesis component and a dehydration (ether forming) component. The reactor system can either be a single three phase reactor or a series of staged three phase reactors. Even though the process of the present invention can be carried out in a series of the staged three phase reactors, the dimethyl ether synthesis is carried out in a single step, i.e. all three reactions in the synthesis route are being driven simultaneously.

The process of the present invention can be operated in either an ebullated bed mode with a granulated (shaped pellet) or a slurry mode with a powdered catalyst. The concentration of catalyst suspended in the liquid medium is in the range from about 5 wt% to about 60 wt%. As stated earlier, the catalyst utilized in the process should comprise both a methanol synthesis component and a dehydration component. The methanol synthesis component can for example comprise a typical copper methanol synthesis catalyst. The dehydration component can be selected from the group consisting of alumina, silica-alumina, zeolites (e.g. ZSM-5), solid acids (e.g. boric acid), solid acid ion exchange resins (e.g. perfluorinated sulfonic acid) and mixtures thereof.

The preferred operating conditions of the process are a pressure range from about 13.78 bar to about 137.8 bar (200 psig to about 2000 psig) more preferably from about 27.56 bar to about 68.9 bar (400 psig to about 1000 psig); a temperature range from about 200°C to about 350°C; and a space velocity in excess of 50 (standard) liter of synthesis gas per kilogram of catalyst, more preferably in the range from about 1000 to about 10,000 (standard) liter of synthesis gas per kilogram of catalyst.

The process is particularly useful for higher CO content synthesis gases, even where the concentration of carbon monoxide in the synthesis gas is in excess of 50 vol%.

The process can also comprise a further step of adding water to the three phase reactor as a co-feed. The water can be added as a liquid or a vapor. The addition of water is particularly benecifical when the concentration of hydrogen in the synthesis gas is less than 10 vol%.

The process of the present invention is particularly suited for use in an integrated gasification combined cycle power plant for the production of electrical energy, wherein the process of the present invention would produce a storable fuel for peak-shaving.

### DETAILED DESCRIPTION OF THE INVENTION

The present invention is a single step process for the direct synthesis of dimethyl ether from synthesis gas with or without co-product methanol in the liquid phase. Selectivity for dimethyl ether and methanol can be optimized by varying reaction conditions and/or catalyst compositions to suit the process application.

The process uses a single catalyst or a physical mixture of catalysts which can be in the form of shaped pellets or in the form of a fine powder, depending on the mode of operation. Many types of catalysts are known in the literature for each individual reaction of the process, and these can be mixed in various proportions in the reactor. Alternately, a single catalyst component may be used to facilitate all three reactions. However, the catalyst utilized in the process should have a methanol synthesis component and a dehydration component. An example of a methanol synthesis catalytic component is copper. Examples of a dehydration or ether forming component are alumina, silica-alumina, zeolites, solids acids such as boric acid, and solid acid ion exchange resins such as perfluorinated sulfonic acids.

Catalyst loadings can vary from very dilute, i.e. 5 wt% of the slurry, to very concentrated, i.e. 60 wt% or higher of the slurry. The catalyst is suspended in an inert oil, such as a paraffinic hydrocarbon or a hydrocarbon blend. Other types of liquids are known to work for liquid phase processes, for example, oxygenated species such as alcohols, ethers, and polyethers. These oxygenated liquids should be inert, and have a boiling point for single component liquids or boiling range for blended liquids between 150°C and 450°C.

In the process of the present invention, synthesis gas is introduced into the reactor and contacts the suspended catalyst. The synthesis gas is typically comprised of H₂, CO, CO₂, and often inert species such as N₂ and CH₄. The composition of the gas can vary widely, as shown in the Examples. Depending on feed concentrations of H₂, CO, and CO₂, it may be advantageous to co-feed H₂O either as a liquid or vapor to the process in order to adjust the gas composition via the shift reaction. This water addition is particularly beneficial when the concentration of hydrogen in the synthesis gas is less than 10 vol%. In addition, it may be advantageous to remove CO₂ from the feed gas in order to affect the dimethyl ether product selectivity. The removal of CO₂ can be accomplished by any conventional means, e.g. pressure swing adsorption or absorption using a CO₂ selective solvent such as amines. The feed gas can be composed entirely of fresh feed in a once-through application, or it may be composed of a mixture of fresh feed and recycled gas.

Process conditions can vary widely, depending on operating conditions and type of reactor. Pressure ranges from ambient to high pressure, since increasing pressure typically enhances synthesis. Preferred pressures range from 13.78 bar to 137.8 bar (200 to 2000 psig), and higher; more preferably 27.56 bar to 68.9 bar (400 to 1000 psig). Temperature can range from 200°C to 350°C, and preferably from 225°C to 300°C. Space velocities, measured as (standard) liter of synthesis gas per kilogram of catalyst per hour can range from 50 to 50,000 and higher, depending on the cost of downstream separation of the dimethyl ether and methanol products; the most preferably range is between 1000 and 10,000 (standard) liter of synthesis gas per kilogram of catalyst per hour.

Process conditions and yields are illustrated in the following series of examples, which describe the use of various catalyst mixtures as well as single components, and co-feed of H₂O. All runs were made in either a 300 cm³ or a 1 liter stainless steel autoclave with feed and product gas analysis via gas chromatograph.

Although the following examples were carried out in a single three phase reactor, the process of the present invention can be carried out in a series of staged three phase reactors. Process conditions for the different reactors can be varied, however, the reactor conditions are not varied from reactor to reactor to isolate and accomplish a single reaction in the synthesis route. The process of the present invention is accomplished by simultaneously carrying out the three reactions of the dimethyl ether synthesis route.

### EXAMPLE I

The first series of experiments were performed with a so-called balanced gas (55% H₂, 19% CO, 5% CO₂, 21% N₂) at 250°C and 55.12 bar (800 psig). A 25 wt% slurry consisting of 20 g BASF S3-85 methanol catalyst and 20 g of 200 mesh Catapal® high purity alumina was prepared in degassed Witco 70 oil. Results are shown in Table I; methanol and dimethyl ether were the only detectable products. Comparison of CO conversions for methanol alone and dimethyl ether shows that the single-step dimethyl ether process is more efficient in overall CO conversion than production of methanol alone. In fact, Runs 1 and 2 show CO conversions greater than the thermodynamic maximum conversion for methanol alone.

CO₂ was found to have a major impact on dimethyl ether formation. Run 5, with no inlet CO₂, showed substantially higher dimethyl ether productivity and selectivity than Run 3. This comparison illustrates the potential for CO₂ removal from the feed gas.

**Table 1**

| Run | Feed | Temp (°C) | GHSV (s-1/kg-catal hr) | Productivity (mol/kg-catal hr) | | DME/MeOH Selectivity (mol%/mol%) | CO Conversion (mol %)¹ |
|---|---|---|---|---|---|---|---|
| | | | | DME | MeOH | | |
| 1 | Bal | 250 | 1500 | 2.3 | 1.6 | 60/40 | 69 (44) |
| 2 | Bal | 250 | 2500 | 3.2 | 2.6 | 55/45 | 55 (40) |
| 3 | Bal | 250 | 2750 | 2.8 | 3.0 | 49/51 | 52 (39) |
| 4 | Bal | 250 | 5000 | 3.6 | 4.2 | 46/54 | 36 (32) |
| 5 | Bal² | 250 | 2750 | 4.7 | 1.5 | 76/24 | 65 |

| | | | | | | | |
|---|---|---|---|---|---|---|---|
| Note 1: Numbers in parentheses indicate carbon monoxide conversion observed for methanol synthesis in the absence of dimethyl ether synthesis. | | | | | | | |
| Note 2: Balanced gas with no inlet CO₂ (57% H₂, 20% CO, 0% CO₂, 23% N₂) | | | | | | | |

### EXAMPLE II

To illustrate the use of a different catalyst mixture, and a different gas feed, a second series of experiments was made with 20 g BASF S3-85 methanol catalyst and 40 g Davison Silica/Alumina in degassed Witco 70 oil. Ten conditions were run with CO-rich and balanced gas at temperatures of 250°C and ∼265°C, and pressure of 55.12 bar (800 psig). Results are shown in Table 2. Balanced gas has the same composition as the earlier examples, and CO-rich gas is comprised of 35% H₂, 51% CO, 13% CO₂, and 1% N₂. Space velocities and productivities are based on the total oxide catalyst charged to the system.

**Table 2**

| Run | Feed | Temp (°C) | GHSV (s-1/kg catal-hr) | Productivity (mol/kg catal-hr) | | DME/MeOH Selectivity (mol%/mol%) |
|---|---|---|---|---|---|---|
| | | | | DME | MEOH | |
| 6 | Bal | 250 | 1870 | 2.46 | 0.96 | 72/28 |
| 7 | Bal | 250 | 3130 | 2.57 | 1.66 | 61/39 |
| 8 | Bal | 250 | 1120 | 1.92 | 0.57 | 77/23 |
| 9 | Bal | 263 | 1870 | 2.57 | 0.92 | 74/26 |
| 10 | Bal | 264 | 3730 | 3.09 | 1.45 | 68/32 |
| 11 | Bal | 265 | 1350 | 2.12 | 0.54 | 80/20 |
| 12 | CO-rich | 250 | 1870 | 1.41 | 0.42 | 77/23 |
| 13 | CO-rich | 250 | 1870 | 1.36 | 0.46 | 75/25 |
| 14 | CO-rich | 250 | 3130 | 1.18 | 0.59 | 66/34 |
| 15 | CO-rich | 250 | 1120 | 1.16 | 0.26 | 82/18 |

### EXAMPLE III

A third series of experiments illustrates operation with H₂O co-feed, and simultaneous shift, methanol and dimethyl ether reactions. A 15 wt% slurry comprised of 25 g BASF K3-110 commercial low-temperature shift catalyst, 25 g BASF S3-85, and 25 g Catapal® alumina was slurried in 425 g degassed Witco 70 oil. The pressure was 55.12 bar (800 psig). The feed gas was 0.8% H₂, 57.7% CO, 15.5% CO₂, and balance N₂. Steam was co-fed with the gas to shift the CO and produce H₂. Results are summarized in Table 3.

**Table 3**

| Run | Feed Ratio H₂O/CO | Temp (°C) | GHSV (s-1/kg-catal hr) | Productivity (mol/kg-catal hr) | | DME/MeOH Selectivity (mol%/mol%) |
|---|---|---|---|---|---|---|
| | | | | DME | MeOH | |
| 16 | 0.50 | 249 | 2000 | 0.40 | 2.19 | 16/84 |
| 17 | 0.33 | 247 | 1860 | 2.64 | 2.11 | 56/44 |

### EXAMPLE IV

A final series of experiments illustrates the use of a single catalyst species in the process. A copper on alumina catalyst was prepared by dissolving 64.03 g Cu(NO₃)₂·2.5H₂O in 100 ml deionized water. The solution was used to impregnate 78.14 g Al₂O₃ in several portions, with N₂ purging between impregnations. The catalyst was dried overnight at 110°C, and reduced with 2% H₂ in N₂. Following reduction, 25 g catalyst (equivilent to 40.6 g as oxide) were slurried in 100 g of degassed Witco 70 oil. The system was run at 55.12 bar (800 psig), and results are shown in Table 4.

**Table 4**

| Run | Feed | Temp (°C) | GHSV (s-1/kg catal-hr) | Productivity (mol/kg catal-hr) | | DME/MeOH Selectivity (mol%/mol%) |
|---|---|---|---|---|---|---|
| | | | | DME | MEOH | |
| 18 | Bal | 249 | 3000 | 0.58 | 0.45 | 56/44 |
| 19 | Bal | 265 | 3000 | 0.82 | 0.41 | 67/33 |
| 20 | Bal | 296 | 3000 | 1.07 | 0.28 | 79/21 |

The present invention with its efficient production of dimethyl ether with a methanol co-product can address a limitation of the liquid phase methanol process, which usually arises in its application for use in integrated gasification combined cycle electric power plants. In this application, the liquid phase methanol process generates methanol, a storable fuel, for peak-shaving. However, the methanol production rate is often limited by thermodynamic constraints. Hence, there is a limit to the amount of storable fuel which can be produced by the liquid phase methanol process. Co-production of dimethyl ether and methanol does not suffer from this thermodynamic constraint. Consequently, an improved process could produce a greater quantity of storable fuel.

The present invention solves the previously described problems through two features inherent in the process. First, the liquid medium acts as a heat sink, resulting in isothermal operation for the reactor. This factor is critical since the forward shift, methanol synthesis, and dimethyl ether synthesis reactions are all exothermic. With conventional gas-phase processes, the heat released during reaction increases temperature, which impedes reaction due to thermodynamic limitations, and causes catalyst deactivation. The high thermal capacity of the liquid phase permits high conversions while maintaining stable temperatures. This excellent temperature control may be responsible for increased catalyst life of this process relative to gas phase operations which require frequent catalyst regeneration.

The second feature of this process is that it uniquely exploits the synergism of the chemistry of all three reactions by combining them in a single step. By combining the three reactions in a simultaneous process, each of the individual reactions is driven thermodynamically by removing its inhibiting products as reactants for the subsequent reaction. For example, the first series of experiments showed CO conversions for the DME process which exceed the thermodynamic maximum for methanol synthesis alone. Such synergism cannot be achieved in multi-step process, where each reaction proceeds at most to its individual thermodynamic limitation in separate reactors.

Also, since all reactions in the process of the present invention proceed co-currently, the process permits several options for varying the product distribution by manipulating the extent of each reaction. For example, the DME/MeOH selectivity can be controlled by varying the amount or activity of catalyst constituents in a process using a mixture of catalysts, or by altering the intrinsic selectivity of the catalyst in a process using a single catalyst. The product distribution can also be varied by changing reaction conditions such as space velocity, temperature, pressure or feed compositions.

As a summary, the distinguishing features of the present invention are the liquid phase operation and the concommitant occurrence of the shift, MeOH synthesis, and DME synthesis reactions. Both features are required for efficient operation and process flexibility.

Although several processes make use of combinations of reactions (1) through (3), the reactions are conducted in the gas phase. Since the shift and methanol synthesis reactions are thermodynamically limited by high temperatures and all three reactions are exothermic, removal of heat from the reactor is a critical and probably the limiting factor in their design. The significance of the thermal control provided by the liquid phase is best illustrated by comparing gas and liquid phase processes. For example, the adiabatic temperature rise for a gas-phase process providing the same conversions as the conditions of Run 1 is 350°C, versus a liquid phase process with an actual temperature rise less than 10°C due to the presence of the liquid phase. No current commercial catalyst could survive the gas phase adiabatic temperature of 600°C, so such a process could not be practically operated without heat removal equipment or product gas recycling. Both options are generally very expensive. For example, using product gas recycling to control the temperature rise would require a recycle ratio in the range of 10 to 20. Such high recycle ratios require high capital investments for compressors and reactors, as well as high operating costs. In comparison, a liquid phase unit would require little or no feed recycle, and a much smaller reactor. Hence liquid phase synthesis can provide economic operation at high conversions.

It should be noted that the prior art has erroneously concluded that liquid phase operation, such as the process of this invention, does not work; Sherwin and Blum failed to produce DME in systems very similar to this process. Hence the success of this process is both novel and surprising. The only other liquid phase synthesis discussed in the prior art is non-selective and consumes an expensive solvent as a reagent, see Daroda et al.

The second distinguishing feature of the invention, the co-current shift, methanol synthesis, and DME synthesis reactions enable the process to use feeds with wide ranges in composition. Previously disclosed processes can only operate within restricted ranges of H₂/CO or CO/CO₂ ratios. This invention demonstrates operation with feeds richer in CO than any previous processes. For example, Runs 16 and 17 show high productivity with feed CO concentrations of 58% and a H₂/CO ratio below 0.02. Such conditions are well beyond those claimed or taught for the prior art processes.

The present invention has been described with reference to several embodiments thereof. These embodiments should not be considered limitations on the scope of the present invention, the scope of which should be ascertained from the following claims.

## Claims

1. A process for the direct synthesis of dimethyl ether from a synthesis gas comprising hydrogen, carbon monoxide and carbon dioxide, wherein the synthesis gas is contacted with and reacted in the presence of a solid catalyst, characterized by contacting and reacting the synthesis gas in the presence of a solid catalyst system, wherein the solid catalyst system is a single catalyst or a mixture of catalysts, suspended in a liquid medium in a three phase (liquid phase) reactor system, wherein the three phase reactor system comprises at least one three phase reactor.

2. The process of Claim 1 wherein the suspended catalyst is in the form of a shaped pellet and the three phase reactor is operated in an ebullated mode.

3. The process of Claim 1 wherein the suspended catalyst is in powdered form and the three phase reactor is operated in a slurry mode.

4. The process of Claim 1 which further comprises operating the process at a pressure in the range from about 13.78 bar to about 137.8 bar (200 psig to about 2000 psig).

5. The process of Claim 1 which further comprises operating the process at a pressure in the range from about 27.56 bar to about 68.9 bar (400 psig to about 1000 psig).

6. The process of Claim 1 which further comprises operating the process at a temperature in the range from about 200°C to about 350°C.

7. The process of Claim 1 which further comprises operating the process at a space velocity in excess of 50 (standard) liter of synthesis gas per kilogram of catalyst.

8. The process of Claim 1 which further comprises operating the process at a space velocity in the range from about 1,000 to about 10,000 (standard) liter of synthesis gas per kilogram of catalyst.

9. The process of Claim 1 wherein the concentration of carbon monoxide in the synthesis gas is in excess of 50 vol%.

10. The process of Claim 1 which further comprises adding water to the three phase reactor as a co-feed.

11. The process of Claim 10 wherein water is added as a liquid.

12. The process of Claim 10 wherein the concentration of hydrogen in the synthesis gas is less than 10 vol%.

13. The process of Claim 1 wherein the concentration of catalyst suspended in the liquid medium is in the range from about 5 wt% to about 60 wt%.

14. The process of Claim 1 wherein the catalyst system comprises a methanol synthesis component and a dehydration (ether forming) component.

15. The process of Claim 1 wherein the catalyst system is a single catalyst containing both a methanol synthesis component and a dehydration (ether forming) component.

16. The process of Claim 14 wherein the methanol component comprises copper.

17. The process of Claim 14 wherein the dehydration (ether forming) component is selected from the group consisting of alumina, silica-alumina, zeolites, solid acids, solid acid ion exchange resins and mixtures thereof.

18. The process of Claim 1 which further comprises removing carbon dioxide from the synthesis gas prior to contacting and reacting the synthesis gas with the solid catalyst.

19. A process for the direct synthesis of dimethyl ether and methanol co-products from a synthesis gas comprising hydrogen, carbon monoxide and carbon dioxide, wherein the synthesis gas is contacted with and reacted in the presence of a solid catalyst, characterized by contacting and reacting the synthesis gas in the presence of a solid catalyst system, wherein the solid catalyst system is a single catalyst or a mixture of catalysts, suspended in a liquid medium in a three phase (liquid phase) reactor system, wherein the three phase reactor system comprises at least one three phase reactor.

20. A process for the generation of electricity by an integrated gasifier combined cycle power plant, wherein a synthesis gas is contacted with and reacted in the presence of a solid catalyst to produce a storable fuel for peak-shaving, wherein the amount of storable fuel is increased, characterized by producing a dimethyl ether co-product in addition to methanol by contacting and reacting the synthesis gas in the presence of a solid catalyst system, wherein the solid catalyst system is a single catalyst or a mixture of catalysts, suspended in a liquid medium in a three phase (liquid phase) reactor system, wherein the three phase reactor system comprises at least one three phase reactor.

## Patentansprüche

1. Verfahren zur Direktsynthese von Dimethylether aus einem Wasserstoff, Kohlenmonoxid und Kohlendioxid umfassenden Synthesegas, worin das Synthesegas mit einem festen Katalysator in Kontakt gebracht und in Gegenwart des Katalysators zur Reaktion gebracht wird, gekennzeichnet durch In-Kontakt-Bringen und Umsetzen des Synthesegases in Gegenwart eines festen Katalysatorsystems, worin das feste Katalysatorsystem ein einzelner Katalysator oder eine Mischung von Katalysatoren ist, der/die in einem flüssigen Medium in einem Dreiphasen-(Flüssigphasen-)Reaktorsystem suspendiert ist/sind, worin das Dreiphasen-Reaktorsystem wenigstens einen Dreiphasenreaktor umfaßt.

2. Verfahren nach Anspruch 1, worin der suspendierte Katalysator in Form eines geformten Pellets vorliegt und der Dreiphasenreaktor im Siedehitzebetrieb betrieben wird.

3. Verfahren nach Anspruch 1, worin der suspendierte Katalysator in Pulverform vorliegt und der Dreiphasenreaktor im Aufschlämmungsbetrieb betrieben wird.

4. Verfahren nach Anspruch 1, welches außerdem die Maßnahme umfaßt, das Verfahren bei einem Druck im Bereich von etwa 13,78 bar bis etwa 137,8 bar (etwa 200 psig bis etwa 2000 psig) zu betreiben.

5. Verfahren nach Anspruch 1, welches außerdem die Maßnahme umfaßt, das Verfahren bei einem Druck im Bereich von etwa 27,56 bar bis etwa 68,9 bar (etwa 400 psig bis etwa 1000 psig) zu betreiben.

6. Verfahren nach Anspruch 1, welches außerdem die Maßnahme umfaßt, das Verfahren bei einer Temperatur im Bereich von etwa 200 °C bis etwa 350 °C zu betreiben.

7. Verfahren nach Anspruch 1, welches außerdem die Maßnahme umfaßt, das Verfahren bei einer Raumgeschwindigkeit über 50 (Standard-)Liter Synthesegas pro Kilogramm Katalysator zu betreiben.

8. Verfahren nach Anspruch 1, welches außerdem die Maßnahme umfaßt, das Verfahren bei einer Raumgeschwindigkeit im Bereich von etwa 1000 bis etwa 10000 (Stan-dard-)Liter Synthesegas pro Kilogramm Katalysator zu betreiben.

9. Verfahren nach Anspruch 1, worin die Konzentration an Kohlenmonoxid im Synthesegas über 50 Vol.-% liegt.

10. Verfahren nach Anspruch 1, welches außerdem den Schritt umfaßt, dem Dreiphasenreaktor als Co-Beschickung Wasser zuzusetzen.

11. Verfahren nach Anspruch 10, worin das Wasser als Flüssigkeit zugesetzt wird.

12. Verfahren nach Anspruch 10, worin die Wasserstoffkonzentration in dem Synthesegas geringer als 10 Vol.-% ist.

13. Verfahren nach Anspruch 1, worin die Konzentration an Katalysator, der in dem flüssigen Medium suspendiert ist, im Bereich von etwa 5 Gew.-% bis etwa 60 Gew.-% liegt.

14. Verfahren nach Anspruch 1, worin das Katalysatorsystem eine Methanolsynthesekompenente und eine Entwässerungs-(Etherbildungs-)Komponente umfaßt.

15. Verfahren nach Anspruch 1, worin das Katalysatorsystem ein einzelner Katalysator ist, der sowohl eine Methanolsynthesekomponente als auch eine Entwässerungs-(Etherbildungs-)Komponente enthält.

16. Verfahren nach Anspruch 14, worin die Methanolkomponente Kupfer umfaßt.

17. Verfahren nach Anspruch 14, worin die Entwässerungs-(Etherbildungs-)Komponente aus der aus Aluminiumoxid, Siliciumoxid-Aluminiumoxid, Zeolithen, festen Säuren, festen sauren Ionenaustauscherharzen und deren Mischungen bestehenden Gruppe gewählt ist.

18. Verfahren nach Anspruch 1, welches außerdem den Schritt des Entfernens von Kohlendioxid aus dem Synthesegas vor dem Kontakt und der Umsetzung des Synthesegases mit dem festen Katalysator umfaßt.

19. Verfahren zur Direktsynthese von Dimethylether und Methanol als Coprodukte aus einem Wasserstoff, Kohlenmonoxid und Kohlendioxid umfassenden Synthesegas, worin das Synthesegas in Kontakt mit einem festen Katalysator gebracht und in Gegenwart des Katalysators zur Reaktion gebracht wird, gekennzeichnet durch In-Kontakt-Bringen und Umsetzen des Synthesegases in Gegenwart eines festen Katalysatorsystems, worin das feste Katalysatorsystem ein einzelner Katalysator oder eine Mischung von Katalysatoren ist, der/die in einem flüssigen Medium in einem Dreiphasen-(Flüssigphasen-)Reaktorsystem suspendiert ist/sind, worin das Dreiphasenreaktorsystem wenigstens einen Dreiphasenreaktor umfaßt.

20. Verfahren zur Erzeugung von Elektrizität mit einem mit einem integrierten Vergaser kombinierten Umlaufkraftwerk, worin ein Synthesegas mit einem festen Katalysator in Kontakt gebracht und in Gegenwart des Katalysators unter Herstellung eines lagerbaren Brennstoffs für das Peak-shaving (Ausgleich von Belastungsspitzen) umgesetzt wird, worin die Menge an lagerbarem Brennstoff erhöht wird, gekennzeichnet durch Herstellen eines Dimethylether-Coproduktes zusätzlich zu Methanol durch In-Kontakt-Bringen und Umsetzen des Synthesegases in Gegenwart eines festen Katalysatorsystems, worin das feste Katalysatorsystem ein einzelner Katalysator oder eine Mischung von Katalysatoren ist, der/die in einem flüssigen Medium in einem Dreiphasen-(Flüssigphasen-)Reaktorsystem suspendiert ist/sind, worin das Dreiphasenreaktorsystem wenigstens einen Dreiphasenreaktor umfaßt.

## Revendications

1. Procédé de synthèse directe de diméthyléther à partir d'un gaz de synthèse comprenant de l'hydrogène, du monoxyde de carbone et du dioxyde de carbone, dans lequel le gaz de synthèse est mis en contact et amené à réagir en présence d'un catalyseur solide, caractérisé par la mise en contact et en réaction du gaz de synthèse en présence d'un système catalyseur solide, le système catalyseur solide étant un catalyseur simple ou un mélange de catalyseurs mis en suspension dans un milieu liquide dans un système réacteur en trois phases (phase liquide), lequel système comprend au moins un réacteur à trois phases.

2. Procédé de la revendication 1 dans lequel le catalyseur mis en suspension se présente sous forme de granulés moulés et le réacteur en trois phases est mis en oeuvre dans un mode d'ébullition.

3. Procédé de la revendication 1 dans lequel le catalyseur mis en suspension est sous forme de poudre et le réacteur en trois phases est mis en oeuvre dans un mode de dispersion.

4. Procédé de la revendication 1 qui comprend par ailleurs la mise en oeuvre du procédé à une pression de l'ordre d'environ 13,78 bar à environ 137,8 bar.

5. Procédé de la revendication 1 qui comprend par ailleurs la mise en oeuvre du procédé à une pression de l'ordre d'environ 27,56 bar à environ 68,9 bar.

6. Procédé de la revendication 1 qui comprend par ailleurs la mise en oeuvre du procédé à une température de l'ordre d'environ 200°C à environ 350°C.

7. Procédé de la revendication 1 qui comprend par ailleurs la mise en oeuvre du procédé à une vitesse spatiale supérieure à 50 litres (standard) de gaz de synthèse par kilogramme de catalyseur.

8. Procédé de la revendication 1 qui comprend par ailleurs la mise en oeuvre du procédé à une vitesse spatiale de l'ordre d'environ 1.000 à environ 10.000 litres (standard) de gaz de synthèse par kilogramme de catalyseur.

9. Procédé de la revendication 1 dans lequel la concentration en monoxyde de carbone du gaz de synthèse est supérieure à 50 % en volume.

10. Procédé de la revendication 1 qui comprend par ailleurs l'addition d'eau au réacteur en trois phases comme co-alimentation.

11. Procédé de la revendication 10 dans lequel l'eau est ajoutée à titre de liquide.

12. Procédé de la revendication 10 dans lequel la concentration en hydrogène du gaz de synthèse est inférieure à 10 % en volume.

13. Procédé de la revendication 1 dans lequel la concentration de catalyseur mis en suspension dans le milieu liquide est de l'ordre d'environ 5 % en poids à environ 60 % en poids.

14. Procédé de la revendication 1 dans lequel le système catalyseur comprend une composante pour la synthèse du méthanol et une composante pour la déshydratation (à l'origine de l'éthérification).

15. Procédé de la revendication 1 dans lequel le système catalyseur est un catalyseur simple contenant une composante pour la synthèse du méthanol et une composante pour la déshydratation (à l'origine de l'éthérification).

16. Procédé de la revendication 14 dans lequel la composante pour le méthanol comprend du cuivre.

17. Procédé de la revendication 14 dans lequel la composante pour la déshydratation (à l'origine de l'éthérification) est choisie dans le groupe composé de l'alumine, de la silice-alumine, des zéolithes, des acides solides, des résines solides échangeuses d'ions acides et de mélanges de ceux-ci.

18. Procédé de la revendication 1 qui comprend par ailleurs l'élimination du dioxyde de carbone du gaz de synthèse avant la mise en contact et en réaction du gaz de synthèse avec le catalyseur solide.

19. Procédé de synthèse directe de diméthyléther et de coproduits de méthanol à partir d'un gaz de synthèse comprenant de l'hydrogène, du monoxyde de carbone et du dioxyde de carbone, dans lequel le gaz de synthèse est mis en contact et amené à réagir en présence d'un catalyseur solide caractérisé par la mise en contact et en réaction du gaz de synthèse en présence d'un système catalyseur solide, le système catalyseur solide étant un catalyseur simple ou un mélange de catalyseurs mis en suspension dans un milieu liquide dans un système réacteur en trois phases (phase liquide), lequel système réacteur en trois phases comprend au moins un réacteur en trois phases.

20. Procédé de génération d'électricité par une centrale électrique a cycle combiné à un gazéificateur intégré dans lequel un gaz de synthèse est mis en contact et amené à réagir en présence d'un catalyseur solide pour produire un combustible à stocker pour la compensation des pointes, ce qui augmente la quantité de combustible à stocker, caractérisé par la production d'un coproduit de diméthyléther en complément du méthanol par mise en contact et en réaction du gaz de synthèse en présence d'un système catalyseur solide dans lequel le système catalyseur solide est un catalyseur simple ou un mélange de catalyseurs mis en suspension dans un milieu liquide dans un système réacteur en trois phases (phase liquide), lequel système réacteur en trois phases comprend au moins un réacteur à trois phases.
